Europäisches Patentamt

European Patent Office

Office européen des brevets

(11) Publication number: **0 297 828**
**A1**

(12) **EUROPEAN PATENT APPLICATION**

(21) Application number: 88305867.9

(51) Int. Cl.⁴: **A61L 15/03**

(22) Date of filing: **28.06.88**

(30) Priority: **29.06.87 US 67985**

(43) Date of publication of application:
**04.01.89 Bulletin 89/01**

(84) Designated Contracting States:
**BE CH DE ES FR GB IT LI NL SE**

(71) Applicant: **THE KENDALL COMPANY**
**One Federal Street**
**Boston Massachusetts 02110-2003(US)**

(72) Inventor: **Charkoudian, John C.**
**41 Eliot Memorial Road,**
**Newton, Massachusetts 02158(US)**
Inventor: **Etheredge III, Robert W.,**
**5 Oak Hill Road**
**Natick, Massachusetts 01760(US)**

(74) Representative: **Kearney, Kevin David**
**Nicholas et al**
**KILBURN & STRODE 30 John Street**
**London, WC1N 2DD(GB)**

(54) **Novel medicated dressings.**

(57) Novel bandage or dressing for topical application of a bioactive reagent, wherein the fabric or pad of the bandage (which may be of per se known construction) is coated or impregnated with a soft, waxy vehicle containing the bioactive reagent.

EP 0 297 828 A1

## NOVEL MEDICATED DRESSINGS

This invention relates to the topical application of medicaments or other bioactive agents to human or animal skin and, more particularly, to a novel system for such applications.

As if well known, the over the counter (OTC) shelves of a typical pharmacy contain an eclectic assortment of tubes, jars and the like of salves and ointments for application to the skin for various effects including, but not limited to analgesia, bacterial action and counterirritancy.

However, application of such ointments and salves is typically messy, non-quantitative and extends beyond the target area where it may contact clothing or other objects.

Various dressings and bandages for the topical application of a medicament are also known and described, for example, in the patent literature.

U.S.P. 3,249,109 issued to Maeth et al discloses a pharmaceutical preparation for topical applications to mucous membranes, particularly as a post-operative dressing following gum surgery, which preparation includes a backing member and a gelatin base containing specified amounts of gelatin, water, pectin, polyhydric alcohol plasticizer and, optionally, a pharmacologically active material, e.g. bactericide, bacteristatic, antacid, anesthetic, hemostat, and others recited in col.2.

U.S.P. 3,339,546 issued to Chen discloses a medicated bandage which can be applied to both internal and external body surfaces, comprising a water impervious film having secured to one surface thereof an adhesive gum-like bonding composition comprising a blend of a water soluble or swellable hydrocolloid admixed with a water insoluble, viscous gum-like elastic binder. As is disclosed in col.2, the bonding composition may incorporate medicaments such as insulin, antibiotics, anesthetics such as benzocaine, anti-inflammatories, etc.

U.S.P. 3,464,413 issued to Goldfarb relates to medical dressings, e.g. surgical dressings, first-aid-type bandages, military medical bandages, operational bandages, and the like, having adhered thereon microscopic discrete rupturable capsules comprising liquid droplets containing a medicament material encapsulated within an outer shell. Disclosed medicaments include medicinals for treating wounds or burns, antiseptics, antibiotics, blood coagulants, local anesthetics, and the like.

U.S.P. 3,598,122 issued to Zaffaroni relates to a medical bandage for the continuous administration of controlled quantities of systemically active drugs by absorption through the skin or mucosa, comprising a sandwich including a backing member, a discrete middle reservoir layer containing the drug confined within a wall member controlling its release rate, and a pressure-sensitive adhesive for contacting the skin. Suitable drugs recited in the paragraph bridging cols. 3-4 include antimicrobial agents, sedatives and hypnotics, psychic energizers, tranquilizers, hormones, androgenic steroids, estrogenic steroids, progestational steroids, thyroxine, antipyretics, antispasmodics, antimalarials, and nutritional agents.

U.S.P. No. 3,632,740 issued to Robinson et al discloses adhesive tapes having a specified adhesive layer containing an anti-inflammatory amount of corticosteroid for treatment of cutaneous lesions.

U.S.P. 3,731,683 issued to Zaffaroni relates to a medical bandage for the controlled release of topically active drugs, comprising a backing, a pressure-sensitive adhesive containing microcapsules of a topically active drug confined within a controlled release wall member. Topically active drugs (col. 6) include antiperspirants, deodorants, astringents, counter irritants, antifungal agents, keratolytics, anti-inflammatory agents and antibacterial agents.

U.S.P. 3,867,520 issued to Mori et al teaches the concept of dissolving or otherwise dispersing a therapeutic agent in a carrier which formsa separate, disperse phase in films which have a continuous matrix of polyamino acid and which tends to migrate to the film surface when the film temperature is raised to body temperature, thereby gradually bringing the therapeutic agent to the film surface for contact with the skin. More specifically, the patent describes and claims a body-contacting solid film for an occlusive dressing consisting essentially of (a) at least one specified amino acid as a solid film-forming matrix; (b) a carrier dispersed in this matrix which is liquid or semi-solid at 37° C and migrates to the surface of the film at that temperature; and (c) a pharmaceutically active agent dispersed in the carrier. Materials which are said to be suitable as carriers or components of carriers for this purpose include (col. 2) oils, fats, and waxes generally and particularly semi-dry and non-drying vegetable oils, animal fats, mineral oil and vegetable animal or mineral waxes. In col. 3 it is stated that the active agent may generally be any biological or chemotherapeutic pharmaceutical agent suitable for topica application, that is, capable of having a medicinal effect on the body. Specifically mentioned are germicides, antifungal agents and hormones. The medicated films are applied directly to the skin and are preferably covered with a gauze to absorb any carrier that migrates to the free (outer) surface of the applied film.

Finally, U.S.P. 4,455,146 issued to Noda et al relates to a plaster comprising a thermoplastic elastomer, an oil or higher fatty acid, a tackifier and a medicinal ingredient. While the plaster is said to exhibit · satisfactory tackiness and adhesive strength when applied to the skin, it is said to be removable without pain or irritation. Useful medicinal ingredients are recited in col. 3.

From the foregoing discussion, it will be seen that the patent literature teaches various formulations, compositions and structures for applying a biologically active agent to the skin. However, for various reasons not known to Applicants, whether they be inoperability, impracticality or cost, Applicants are not aware of any of these products ever having found commercial acceptance.

The present invention is directed to be a simple but elegant system for efficaciously releasing a medicament to the target area of the skin on demand, which system utilizes well known bandage structures.

In accordance with the present invention the absorbent woven or nonwoven component of a bandage or dressing of per se known construction, e.g. a sterile bandage of the type commonly used for wound dressings or an adhesive bandage such as the type known variously as first-aid bandages, strip bandages or finger bandages, is coated or impregnated with a vehicle containing an effective amount of a medicament or other bioactive reagent, the vehicle comprising a soft, waxy material which is solid at room temperature but which has a low melting point, e.g. below 40°C and which will release the medicament to topical application rapidly following contact with the skin, i.e. rapidly after the bandage is applied.

In the preferred embodiment, the bandage is intended for use as a dressing for an injury to the skin and the medicament for topical application is one that will promote healing and/or provide general comfort for the user, e.g. an anesthetic, a counterirritant, a fungicide or a bactericide.

As previously mentioned, the present invention relates to bandages of per se known construction and which will release a topical medicament to a target area, namely to a skin laceration, irritation, burn, insect bite or other injury rapidly after application. The medicaments contemplated for release in accordance with the invention include but are not limited to those typically sold over the counter in tubes, jars and the like for first-aid treatment. In this context, the present invention may be regarded as a unitary dressing combining the sterile protective covering for the inuured area with one or more medicaments which are heretofore applied separately as liquids, ointments or salves.

The bandage pad material may be any of the woven or nonwoven fabrics of natural or synthetic fibres heretofore employed as dressings. It may and prefer ably will be an adhesive bandage such as the finger bandages commonly employed for the first aid treatment of minor injuries.

In its broadest aspect, therefore, the present invention may be said to relate to the impregnation or coating of a bandage pad material with a medicament-containing vehicle for application to an injured area of the skin. Upon application to the target site, the impregnated bandage releases the active ingredient for treatment. Whereas in the past the application of such topical medicaments required the use of messy, non-quantiative and easily erodible ointments, salves and liquids, the present invention provides a dimensionally stable structure so that the medicament is conveniently released to the target on demand.

The vehicles for delivering the medicament in accordance with this invention are soft, waxy, or wax-like materials having a low melting point, e.g. below 40°C (104°F) and which do not spread at room temperature and in absence of moisture or other liquids, e.g. sebum or skin oils. As used herein, the term "waxy" connotes a substance likened to or resembling wax as soft, impressionable or readily moulded (as defined in Webster's Dictionary) and is not restricted to the chemical definition of waxes, i.e. esters of fatty acids and alcohols other than glycerol. In addition to waxes of animal or vegetable origin within the above chemical definition, mention may be made of waxy materials such as polyethylene glycol (a polyglycol derived from ethylene glycol), e.g. polyethylene glycol 1000, 1450, 1500 or 1540, including mixtures thereof, lanolin, petrolatum, synthetic waxes, etc.

Particularly useful in the practice of this invention are the aforementioned polyethylene glycols. They absorb water from the skin almost instantly, thereby further lowering the melting point.

The medicaments which may be dispersed or carried by these waxy vehicles may in general be any of the medicaments typically applied topically to wounds, burns or other injuries, e.g. those disclosed in the aforementioned patents. By way of illustration, mention may be made of anesthetics such as benzocaine; keratolytics such as salicylic or benzoic acid; anti-inflammatory agents such as the corticosteroids, e.g. hydrocortisone or hydrocortisone acetate; antibacterial agents such as bacitracin or benzalkonium chloride, or neomycin; and other topical medicaments such as those found over the counter performing their particular desired functions.

These medicaments may be incorporated simply by heating the waxy vehicle to liquify it and then dissolving or dispersing the medicament.

The percentage of medicament to vehicle will of course vary in accordance with the particular medicaments employed and is accordingly not capable of precise quantitative definition. However, in general it may be said that on the order of 5-15% by weight of medicament, based upon the total weight of

3

the topical preparation, may be employed.

The waxy vehicle for topical application of a medicament in accordance with this invention may include other materials. A particularly useful additive for this purpose is polyvinylpyrrolidone (PVP), e.g. in a ratio of 4:6 to 6:4 by weight of K90 grade PVP to the waxy material. PVP is water-soluble, but when admixed with polyethylene glycol, for example, becomes very tacky until it absorbs water. This tackiness may tend to provide increased contact of the topical formulation to the skin. However, when water is absorbed from the skin, it instantly becomes very fluid and non-tacky, thereby releasing the polyethylene glycol containing the medicament. As examples of other materials which may be incorporated, mention may be made of polyvinyl alcohol, hydroxyethyl cellulose, carboxymethyl cellulose, starch, mono- and disubstituted polyethylene glycols, copolymers of n-vinylpyrrolidone, reagents such as penetration enhancers, etc.

The waxy formulations carrying the medicament to be applied may be either wicked or coated onto the known woven or nonwoven bandage materials. They may, for example, be incorporated in thebandage as a solution in a volatile, organic solvent, e.g. methanol, ethanol, cyclohexane, acetone, diethylether, hexane, or heptane, in which case the bandage is impregnated with the solution of waxy material, medicament and any addenda, followed by solvent evaporation.

In lieu of utilizing a volatile solvent, the waxy formulation may simply be melted and then wicked into or coated onto the bandage material.

In either case, they form waxy platforms on the bandage substrate which, as detailed hereinafter, release the active medicament for topical application within very few seconds after the bandage is placed on the target area.

Of particular interest in this invention are the application of anesthetics such as benzocaine for local pain or discomfort; hydrocortisone for itching, e.g. for insect bites; and salicylic acid for skin disorders such as acne disorders.

Woven and nonwoven fabrics which may be employed for the bandage substrate in the practice of this invention span a large range of wetting and wicking fabrics and combinations of fibres may be utilized, depending upon the particular formulations employed.

For optimum results, the bandage fabric will be selected for maximum retention of the topical formulation. For example, if a hydrophilic formulation is used, best results are obtained by selecting a polar bandage material such as cotton, rayon or a hydrophilic polyester to obtain maximum wetting and wicking of the formulation. On the other hand, if a hydrophobic formulation is used, polyethylene, polypropylene, or hydrophobic polyester fabrics will in general provide optimum retention.

From a practical manufacturing standpoint, the present invention is commercially feasible in the sense that mass production from commercially available materials is readily accomplished. For instance, one may utilize on a commercial scale the continuous impregnation of rolls of fabric from per se known coating systems, followed by slitting or cutting into individual bandages. It is also contemplated from a manufacturing standpoint that the bandage material be cut and placed on the dressing backing, e.g. the adhesive surface of a medical grade or skin adhesive tape, followed by metering of the topical formulations onto the free surface of the pad material.

The latter system is particularly efficacious in the manufacture of finger bandages carrying the medicament, e.g. an anesthetic, to be released on a minor wound site to alleviate discomfort.

The inventin may be put into practice in various ways and a number of specific embodiments will be described to illustrate the invention with reference to the accompanying examples.

EXAMPLE 1

80 grams of polyethylene glycol, average molecular weight = 1450 are melted at 50°C and 20 grams of benzocaine are then introduced with stirring to form a clear solution. The resulting solution was then coated onto "WEBRIL R" (a trademark of The Kendall Company for a 100% cotton nonwoven) at a rate of 120 grams/square yard to provide approximately 200 square inches of impregnated nonwoven. This coated nonwoven had a visually somewhat glossy, waxy-feeling surface with a portion of the coating solution impregnating or entering the interstices between the fibres. The coated nonwoven was then cut into "finger bandage" dimensions (approximately 1" x 0.75") (2.5 x 1.9 cms) and centrally positioned on the coated surface of a 3" (7.6 cms) medical-grade adhesive strip of the type employed on a "CURAD" (trademark of The Kendall Company) finger bandage, comprising an acrylic adhesive carried on a highly perforated polyester backing and, in conventional manner, a pair of protective overlapping release sheets were applied over the resulting nonwoven and free adhesive to provide a finger bandage of known construction, except for the benzocaine-containing waxy vehicle.

Upon application of the resulting bandage to the skin, benzocaine was very rapidly transferred from the bandage to the skin, as will be more apparent from the transfer rate studies which are described below.

## EXAMPLE 2

40 grams of polyvinylpyrrolidone (PVP), grade K90, 60 grams of polyethylene glycol 400 and 25 grams of benzocaine were dissolved in 125 ml of methanol by rolling the ingredients on a jar mill overnight. After removal of air bubbles, the resulting liquid was applied and allowed to infuse into the nonwoven described in Example 1. The methanol solvent was then removed by air drying under mild (50°C) heat. The resulting impregnated nonwoven containing 20% by weight of benzocaine (based upon the total solids content) was observed to be very tacky to the feel but was stable to heat (100°C). A finger bandage was prepared from the impregnated nonwoven in a manner described in Example 1. Upon contact with the skin it lost its tackiness and rapidly became fluid, releasing the benzocaine for topical application. In view of the initial tackiness, the bandage was noted to have excellent contact with the skin, thereby speeding the benzocaine delivery.

Upon application of the finger bandages of this invention to the skin, benzocaine is very rapidly transferred to the desired area, e.g. a wound. This can be demonstrated, for example, by washing the target site 15-30 seconds after the application and analyzing the wash solution for benzocaine. In addition, several reapplications of the same bandage to the washed target site demonstrated that substantially equivalent further transfers occurred, demonstrating that the waxy vehicle matrix will deliver benzocaine over long duration.

Quantitative evaluations were made of the finger bandage of Example 1 over varying time periods up to one hour.

The approximate mass of benzocaine delivered topically was as follows:

| Time (Minutes) | Mass (ug) |
|---|---|
| 2.5 | 32 ± 6 |
| 5.0 | 47 ± 12 |
| 10.0 | 75 ± 5 |
| 60.0 | 97 ± 15 |

While no quantitative analyses were made at less than 2.5 minutes (as noted above) subjective in vivo tests confirm the rapidity of transfer to the skin. Specifically, this is confirmed by placing the bandage of Example 1 in contact with the lip. In only a few seconds, a slight numbing sensation is experienced.

Studies were also made comparing the mass of benzocaine delivered transdermally from the finger bandage of Example 1 with the amount delivered from an applied commercially available benzocaine ointment.

The approximate amounts delivered transdermally with the finger bandage of Example 1 are:

| Time (Minutes) | Mass (ug) |
|---|---|
| 10 | 2 ± 1 |
| 20 | 10 ± 6 |
| 30 | 24 ± 12 |
| 40 | 42 ± 23 |
| 50 | 64 ± 34 |
| 60 | 87 ± 44 |

As distinguished therefrom, the commercial ointment vehicle delivered less than 10 ug in 30 minutes;

approximately 20 ug in 50 minutes and about 30 ug in 60 minutes, demonstrating the marked superiority of the waxy matrix to the commercial vehicle. In this context, it will further be noted that the ointment, like any ointment would typically be, was applied in a much thicker layer, thereby establishing, as was anticipated, that it is the efficiency of delivery from the vehicle which is most critical, not the quantity applied.

The finger bandages of Example 1 were subjected to shelf life stability testing by storing at 120°F (49°C) over a period of three months. No significant deterioration in construction was noted after aging.

In the foregoing description emphasis has been placed upon the preferred contemplated usage as a medicament incorporated in a bandage of the type commonly used as a first-aid dressing, e.g. as a protective cover for a minor wound, insect bite or other minor skin irritation. As will be appreciated, in addition to providing a sterile protective cover, it is also frequently desirable to alleviate the minor discomfort commonly associated with such invasions to the skin. For this purpose, ointments and salves are commercially available. However, it will be appreciated that the use of such medicaments is messy, awkward and, moreover, requires an addition to the first-aid kit. The present invention provides an elegant alternative for delivering the requisite amount of medicament on demand to the target site where the bandage is applied.

It will also be apparent to those skilled in the art that the present invention is also applicable to other topical applications, e.g. to delivering medicaments or other bioactive agents for purposes other than first-aid treatment of minor wounds and such other topical applications are accordingly contemplated.

Since certain changes may be made without departing from the scope of the invention herein involved, it is intended that all matter contained in the above description shall be interpreted as illustrative and not in a limiting sense.

## Claims

1. A bandage adapted for placement on the skin for topical application of a bioactive reagent comprising at least a fabric pad coated or impregnated with a matrix vehicle containing the said bioactive reagent, the said vehicle comprising a soft, waxy material which has a low melting point and, in the absence of liquids, does not spread at room temperature, but which will rapidly release the said contained bioactive reagent upon contact with the skin.

2. A bandage as claimed in Claim 1 in which the said reagent is selected from the group consisting of anesthetics, counterirritants, fungicides and bactericides.

3. A bandage as claimed in Claim 1 or Claim 2 which includes adhesive means for adhering said bandage to the skin.

4. A bandage as claimed in Claim 1, 2 or 3 in which the said fabric is a nonwoven.

5. A bandage as claimed in any one of Claims 1 to 4 in which the said bioactive reagent is benzocaine.

6. A bandage as claimed in any one of Claims 1 to 5 in which the matrix vehicle is characterised by having a melting point below 40°C.

7. A bandage as claimed in any one of Claims 1 to 6 in which the matrix vehicle comprises polyethylene glycol.

8. A bandage as claimed in Claim 7 in which the said matrix vehicle further includes polyvinylpyrrolidone.

9. A bandage as claimed in Claim 8 in which the said polyvinylpyrrolidone is K90 grade.

10. A bandage as claimed in Claim 8 or Claim 9 in which the said polyvinylpyrrolidone is present in a ratio to polyethyleneglycol of from about 4:6 to 6:4 by weight.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,X | US-A-3 249 109 (H. MAETH et al.)<br>* Column 2, lines 28-46; example 1 * | 1-5 | A 61 L 15/03 |
| D,X | US-A-3 339 546 (J. LING CHEN)<br>* Column 2, lines 5-25; column 2, lines 55-61; example 7 * | 1-5 | |
| A | FR-A-1 474 448 (T.J. SMITH & NEPHEW LTD)<br>* Claims * | | |
| X | GB-A-1 038 532 (ROUSSEL-UCLAF)<br>* Page 1, lines 8-14; page 1, lines 63-67; example 7 * | 1 | |
| A | US-A-4 307 075 (F.H. MARTIN)<br>* Column 3, lines 38-41; column 4, lines 1-4; example 2; claim 1 * | 1-8 | |
| A | EP-A-0 206 697 (JOHNSON & JOHNSON PRODUCTS INC.)<br>* Page 9, lines 17-37; claims * | | TECHNICAL FIELDS SEARCHED (Int. Cl.4)<br><br>A 61 L |

The present search report has been drawn up for all claims

| Place of search | Date of completion of the search | Examiner |
|---|---|---|
| THE HAGUE | 30-09-1988 | ESPINOSA Y CARRETERO M. |

EPO FORM 1503 03.82 (P0401)